# EUROPÄISCHE PATENTSCHRIFT

(11) **EP 1 140 005 B1**
(45) Veröffentlichungstag und Bekanntmachung des Hinweises auf die Patenterteilung: **16.02.2005**
(21) Anmeldenummer: 99965482.5
(22) Anmeldetag: 16.12.1999
(51) Int. Cl.: A61K 7/48, A61K 7/06, A61K 35/78

(54) **VERWENDUNG EINES EXTRAKTES EINER PFLANZE DER CECROPIA-GATTUNG**
UTILIZATION OF AN EXTRACT OF A PLANT OF THE CECROPIA GENUS
UTILISATION D'UN EXTRAIT D'UNE PLANTE APPARTENANT AU GENRE CECROPIA

(30) Priorität: 21.12.1998 FR 9816289
(43) Veröffentlichungstag der Anmeldung: 10.10.2001
(73) Patentinhaber: Cognis France S.A., 31360 Saint-Martory (FR)
(72) Erfinder: PAULY, Gilles, F-54000 Nancy (FR); MORETTI, Christian, F-75010 Paris (FR)
(86) Internationale Anmeldenummer: PCT/EP1999/010031
(87) Internationale Veröffentlichungsnummer: WO 2000/037038

(56) Entgegenhaltungen:
- US-A- 2 871 235
- STN, Serveur de Bases de Données, Karlsruhe, DE, Fichier Chemical Abstracts, Vol 91, AN=16729 * résumé * XP002115142
- STN, Serveur de bases de Données, Karlsruhe, DE, Fichier Chemical Abstracts, Vol 79, AN=50748 * résumé * XP002115143
- STN, Serveur de Bases de Données, Karlsruhe, DE, Fichier Chemical Abstracts, Vol 93, AN=31618 * résumé * XP002115144
- Grenand P., Moretti C., Jaquemin H., "Pharmacopées traditionelles en Guyane: Créoles, Palikur, Wayapi", Orstom-Verlag 1987, S.315-316
- Grenand P.; Moretti C.; Jaquemin H.: 'Pharmacopées traditionelles en Guyane: Créoles, Palikur, Wayapi', Orstom-Verlag 1987, S.315-316

## Beschreibung

Die vorliegende Erfindung betrifft das Gebiet der Kosmetik, besonders der Dermo-Kosmetik, mit der Zielsetzung mindestens einen Extrakt einer Pflanze der Cecropia-Gattung in der Kosmetik, sowie ein kosmetisches oder dermopharmazeutisches Erzeugnis zu verwenden, das mindestens einen derartigen Extrakt enthält.

Die Cecropia-Gattung (MORACEAE) beinhaltet ungefähr 100 schnell wachsende Baum- oder Buscharten, die in ganz Mittelamerika angetroffen werden und von denen einige in traditionellen Medizinbehandlungen verwendet werden.

In Französisch-Guyana werden besonders die Cecropia obtusa Trecul-, Cecropia peltata-, Cecropia shreberiana-, Cecropia palmata-, Cecropia obtusa-Arten vorgefunden, wobei die letztere die am meisten verbreitete ist.

Cecropia obtusa, sowie andere Cecropia ähnlicher Art finden in ganz Mittelamerika Anwendung als Heilpflanzen.

Diese Verwendungen als Heilpflanzen erfolgen entweder in der Form externer Anwendungen, besonders in der Behandlung von Brüchen, dem Aufsaugen (der Resorption) von Blutergüssen (Hämatomen), dem Vernarben von Wunden, zum Desinfizieren der Genitalien und zur Schmerzlinderung nach Entbindungen (siehe "Pharmacopées traditionnelles en Guyane : Créoles, Palikur, Wayapi" GRENAND P., MORETTI C., JAQUEMIN H., Orstom-Verlag, 1987) oder durch Schlucken zur Behandlung von gastrischen oder Darm-Erkrankungen oder auch von Kopfschmerzen (siehe BARRETT B.: "Medicinal Plants of Nicaragua's Atlantic coast.", Economic Botany, 48, 8-20, 1994 / CACERES A. und Koll.: "Plants used in Guatemala for the treatment of gastrointestinal disorders. I. Screening of 84 plants against enterobacteria." J. Ethnopharmacol., 30, 55-73, 1990).
Aus Studien gingen verschiedene chemische Zusammensetzungen in Pflanzen der Cecropia-Gattung klar hervor.

So enthalten die Blätter Kardenolide, Flavonoide, Leukoanthocyanine, Triterpene, Tanine und Polyphänole und die Rinde enthält Steroide, und Ursolinsäure.

In der ganzen Pflanze wurde das Vorkommen von Ambain und Cecropin festgestellt, die kardiotonische und harntreibende Eigenschaften haben.

Ausserdem enthalten die Müller-Cecropia peltata-Substanzen ein Polysaccharid, dessen Struktur der des Glykogens nahe zu sein scheint und es wurde schon vorgeschlagen (US 2 871 235) Polysaccharidstoff ausgehend von den Pflanzen der Cecropia-Gattung zu präparieren.

Aber die Autoren der vorliegenden Erfindung haben auf unerwartete und erstaunliche Weise festgestellt, dass Extrakte aus Pflanzen der Cecropia-Gattung eine gewisse Anzahl von vorteilhaften Eigenschaften und eine sehr gute Toleranz aufweisen, die ihre Verwendung in der Kosmetik und Dermatologie interessant machen, besonders wegen ihrer besonderen und verstärkten Alktivitäten auf die Lipolyse, die sie nützlich in Präparationen für Schlankheitskuren machen, aber auch wegen ihrer straffenden Wirkung, ihrer glättende Eigenschaften und der Verbesserung des strahlenden Glanzes der Haut

Deshalb betrifft das Hauptziel der vorliegenden Erfindung die Verwendung mindestens eines Extraktes einer Pflanze der Ceropia-Gattung als Wirkstoff, alleine oder in Verbindung mit mindestens einem anderen Wirkstoff für die Präparation eines Erzeugnisses oder einer kosmetischen oder dermopharmazeutischen Zusammensetzung für lokale, äussere Anwendung für die Haut, die Schleimhäute und/oder die Epithel- oder Körperanhanggebilde (Haare, Nägel, ...) gemäß der Ansprüche 1 und 6.

Nach einer bevorzugten Ausführungsart der Erfindung kann das Wirkungsmittel durch einen Extrakt der Cecropia obtusa und/oder Cecropia peltata-Pflanze/n gebildet werden, wobei die für die Präparation des Extraktes oder der Extrakte verwendeten Teile der Pflanzen die Wurzeln, die Rinden (der Wurzeln, der Stengel und des Stammes), die Blätter und beblätterten Stengel, die Früchte, die Körner (Samen) und/oder die Blüten sind.

Das für die Ausführung des Extraktes oder der Extrakte verwendete Lösemittel wird in der Gruppe gewählt, die durch Wasser, Alkohole, Ketone, Ester, Äther, Polyole, Chlorlösemittel und Gemischen von mindestens zweien der vorab erwähnten Lösemittel gebildet wird.

Aber die Extraktion kann auch mit superkritischem CO₂ alleine oder im Gemisch mit einem Beilösemittel stattfinden.

Nach einer besonderen Ausführungsart der Erfindung wird der Extrakt durch ein Verfahren gewonnen, das auf einer Wellenbestrahlung, wie zum Beispiel Mikrowellen oder Ultraschall basiert.

Nach einer ersten Alternative der Ausführung der Erfindung ist der verwendete Extrakt ein totaler Cecropia-Extrakt, der alle aktive, extrahierbare Bestandteile der Pflanze enthält.

Nach einer zweiten Alternative der Ausführung der Erfindung wird der Extrakt durch eine Fraktion gebildet, die rohe, halb gereinigte oder gereinigte Polysaccharide enthält.

Nach einer dritten Alternative der Ausführung der Erfindung wird der Extrakt aus einer Fraktion gebildet, die aktive Substanzen der Pflanze/n enthält, mit Ausnahme der Polysaccharide.

Nach einer Alternative einer vorteilhaften Ausführung der Erfindung wird der Extrakt durch eine Fraktion gebildet, die mindestens halb gereinigt ist, zum Beispiel durch Chromatographietechnik oder einer fraktionierten flüssigen/flüssigen oder flüssigen/kompakten Extraktionstechnik.

Zur Veranschaulichung, aber nicht zur Einschränkung werden nachstehend verschiedene Verfahren beschrieben, die vorzugsweise für die Ausführung der Extrakte aus Pflanzen der Cecropia-Gattung angewendet werden können, die direkt im Rahmen dieser Erfindung verwendbar sind.

Es sollte beachtet werden, dass der in der Beschreibung der Beispiele verwendete Teil der Pflanze als Angabe erwähnt wird, wobei die Extrakte, die Gegenstand der Erfindung sind, aus allen zugänglichen Teilen der vorab erwähnten Pflanzen ausgeführt werden können.

### Beispiel 1

Rinden des Cecropia obtusa-Stammes werden grob zerkleinert und dann im Messerzerquetscher fein gequetscht.

In einen mit einem Rührer versehenen Reaktor werden 3,75 Liter destilliertes Wasser geleitet und danach werden die folgenden Schritte vorgenommen, die bestehen aus:
- dem Hinzufügen von 300 g zerquetschter Rinde in den Reaktor,
- dem Homogenisieren der zähflüssig gewordenen Lösung durch Ultra-Turrax,
- dem Extrahieren unter Rühren während einer Stunde bei Sieden (85-90° C),
- dem Abkühlen auf Raumtemperatur,
- dem Beseitigen der unlösbaren Stoffe durch Zentrifugieren bei 5000 g,
- dem Auffangen der an der Oberfläche schwimmenden Stoffe, woraus sich 2,07 Liter E1-Extrakt zu 1,1 % trockener Materie ergeben,
- dem Extrahieren des Zentrifugierbodensatzes unter den gleichen Betriebsbedingungen durch 2,75 Liter destilliertes Wasser und danach wie vorhergehend arbeiten, um 2,6 Liter E2-Extrakt zu 0,71 % trockener Materie zu gewinnen.

Der gesamte Ertrag der beiden Extraktionen, der auf der Basis des trockenen Extraktes bestimmt wird, beträgt 13,7 % / Rinden.

Die gewonnenen Lösungen werden dann durch Zerstäubung oder jegliche andere Technik entwässert (deshydratisiert), die dem Fachmann auf diesem Gebiet bekannt ist, (Gefriertrocknung - Lyophilisierung, Trocknen im Trockenofen usw.).

In diesem Beispiel wird ein Liter E1-Extrakt (ohne Zufügen eines Zusatzes) zerstäubt und durch diesen Vorgang werden 7,4 g Pulver gewonnen, das bedeutet ein Zerstäubungsertrag von 65,9 %.

### Beispiel 2

Ein Liter zähflüssiger, durch das Beispiel 1 gewonnener Extrakt wird unter Rühren in einen Liter Alkohol, 96°, hinzugefügt, um die enthaltenen Polysaccharide zu fällen.

Die gebildeten Fasern werden geschleudert und bei Raumtemperatur in 150 ml Äthanol während mindestens einer Stunde getaucht.

Die Fasern werden dann in 200 ml Aceton während einer Stunde getaucht, geschleudert und danach im bei 40° C belüfteten Trockenofen getrocknet.

Das Gesamtgewicht der gewonnenen Polysaccharide beträgt 9,5 g, das bedeutet eine theoretische Ausbeute, wenn der ganze Extrakt des Beispiels 1 von 6,5 % Rinden gefällt wurde:

### Beispiel 3

In einen mit einem Rührer versehenen Reaktor werden 2,2 Liter 50 %iger Äthylalkohol (Äthanol oder Ethanol) geleitet und danach werden die folgenden Schritte unternommen, die bestehen aus:
- dem Hinzufügen von 300 g Cecropia obtusa-Rinde, die nach dem Beispiel 1 im Reaktor zerquetscht wurde,
- dem Extrahieren unter Rühren während einer Stunde unter Rückfluss,
- dem Abkühlen auf Raumtemperatur,
- dem Filtern des an der Oberfläche schwimmenden Stoffs bis auf eine Porosität von ungefähr 0,45 µm,
- dem Auffangen des braunen Filtrats,
- dem Beseitigen des Restes (unlöslich) durch grobes Zentrifugieren oder Filtern,
- dem Verdampfen der Alkoholphase des Filtrats unter Vakuum, um 667 ml wässrige Lösung zu gewinnen, deren Gehalt an gelösten Stoffen 1,59 % beträgt (das bedeutet eine Extraktionsausbeute von 3,53 % / Rinden),
- dem Hinzufügen eines Zusatzes des Maltodextrintyps in die Lösung, um 2/3 Zusatz für 1/3 extrahierter trockener Materie zu gewinnen,
- dem Entwässern der gewonnenen Lösung durch Zerstäubung,
- dem Extrahieren des Restes unter den gleichen Betriebsbedingungen durch 2,25 Liter 50 %iges Äthanol und die verschiedenen vorhergehenden Schritte wiederholen, um 875 ml wässrige Lösung zu gewinnen, deren Gehalt an gelösten Stoffen 0,81 % beträgt (das bedeutet einen Ertrag von 2,36 % / Rinden),
- dem Zerstäuben der Lösung nach dem Hinzufügen von Maitodextrin zu den gleichen Bedingungen wie vorhergehend.

Der gesamte theoretische Ertrag der Extraktion beträgt 5,89 % / Rinden.

### Beispiel 4

In einen mit einem Rührer versehenen Reaktor werden 3,37 Liter destilliertes Wasser geleitet und dann unter Rühren 270 Gramm getrocknete Restmaterie (unlöslich), die nach der Vorbereitung des Extraktes des Beispiels 3 gewonnen wurde.

Danach werden aufeinanderfolgend die Schritte vorgenommen, die bestehen aus:
- dem Rühren bis zur Löslichkeit und danach Homogenisieren des zähflüssig gewordenen Gemischs durch Ultra-Turrax,
- dem Ansteigen der Temperatur auf 80-85° C,
- dem Extrahieren während einer Stunde unter Rühren,
- dem Abkühlen auf 30° C,
- dem Zentrifugieren während 15 Minuten bei 5 000 g,
- dem Auffangen des an der Oberfläche schwimmenden braunen, zähflüssigen Stoffs (2 Liter),
- dem Fällen der im Extrakt vorhandenen Polysaccharide durch das Hinzufügen in ein Volumen Äthanol, 96°, und danach wie im Beispiel 2 verarbeiten.

Auf diese Weise werden 12,7 Gramm Polysaccharide gewonnen, das heisst ein Ertrag von 4,70 % im Verhältnis zur Restmaterie und von 4,2 % / Rinden.

### Beispiel 5

In einen Reaktor werden 2,25 Liter Äthylalkohol, 96°, geleitet und danach werden aufeinanderfolgend die Schritte vorgenommen, die bestehen aus:
- dem Hinzufügen, in den Reaktor, von 300 g Cecropia obtusa-Rinde, die nach dem Beispiel 1 zerquetscht wurde,
- dem Extrahieren unter Rühren während einer Stunde unter Rückfluss,
- dem Abkühlen auf Raumtemperatur,
- dem Beseitigen der ungelösten Materie durch Filtern,
- dem Filtern des gewonnenen Extraktes bis auf eine Porosität von ungefähr 0,45 µm,
- dem Auffangen des Filtrats,
- das Lösungsmittel unter Vakuum bei 40° C verdampfen lassen,
- dem Beseitigen der letzten Spuren des Lösungsmittels im Trockenofen bei 50° C.

Das Gewicht der gewonnenen Extrakte beträgt 4,07 Gramm, das heisst ein Ertrag R1 = 1,35 % / Rinden.

Die Aufbereitung der Restmaterie unter den gleichen Betriebs-bedingungen mit 2,25 Liter Alkohol ermöglicht 2,41 Gramm Extrakte zu gewinnen, das heisst, ein Ertrag R2 = 0,80 % / Rinden.

### Beispiel 6

In einen mit einem Flügelrührer versehenen Reaktor werden 3,37 Liter destilliertes Wasser geleitet.

Danach werden aufeinanderfolgend die Schritte vorgenommen, die bestehen aus:
- dem Einleiten unter Rühren von 270 Gramm getrocknete Restmaterie (unlöslich) der Extraktion des vorab erwähnten Beispiels 5,
- dem Rühren bis zur Löslichkeit, Homogenisieren durch Ultra-Turrrax und dann wie im Beispiel 4 vorgehen.

Das Gesamtgewicht des gewonnenen Pflanzenschleims (Mucilago) beträgt 15,54 Gramm, das heisst ein Ertrag R1 = 5,75 % im Verhältnis zur Restmaterie und 5,18 % / Rinden.

Jetzt werden die Tests zum Nachweis und Auswertung der von den Erfindern entdeckten Haupteigenschaften beschrieben, die Pflanzen-extrakte der Cecropia-Gattung betreffen.

### 1) Nachweis der Aktivierungswirksamkeit der Lipolyse auf menschliche Adipozyte im "in vitro"-Überleben

Nachstehend wird der Test beschrieben, der für die Hervorhebung der Aktivierung der Lipolyse der menschlichen Adipozyten bei Vorhandensein von Cecropia-Extrakten verwendet wurde, die Gegenstand der vorliegenden Erfindung sind.

Die Lipolyse ist die Ausscheidung der Triglyzeride (oder TG), die in den Adipozyten gespeichert sind, durch ein hormon-sensibles Enzym: die Triglyzerid-Lipase (oder TGL), die die TG in freie Fettsäuren und Glyzerin spaltet, die im Blutkreislauf ausgeschieden werden. Die Fettsäuren können dann durch die Muskelzellen aufgefangen werden, um Energie zu erzeugen.
1) Prinzip des in vitro-Tests
   Die Aktivierung der Lipolyse wird durch spektrophotometrische Dosierung der Rate des ausgesalzenen Glyzerins durch "in vitro" inkubierte Adipozyten bei Vorhandensein der zu testenden Substanz ausgewertet.
   Die Adipozyte werden durch enzymatische Verdauung des subkutanen menschlichen Gewebes nach der sogenannten Rodbell-Technik isoliert (siehe M. Rodbell: "Metabolism of isolated fat cells". The journal of biological chemistry, Band 239, N° 2, Seite 375 bis 380, 1964).
2) Aufbereitung der Adipozyte in Aufschwemmung (Suspension)
   Die Cecropia-Extrakte werden in einem definierten Medium aufgelöst, das 90 Minuten lang bei 37° C mit den Adipozyten in Kontakt gebracht wird.
3) Mengenbestimmung (Quantisierung) der Lipolyse
   Die Rate des ausgesalzenen Glyzerins wird durch Spektrophotometrie im Medium der an der Oberfläche schwimmenden Stoffe nach der von C. Carpéné und Koll. beschriebenen Technik "Discrimination des adrénergiques à potentialité α2 par l'étude de la lipolyse sur des adipocytes de hamster". (J. Phamacol. (Paris), Band 12, N° 2, Seite 219 bis 224, 1981) mengenbestimmt.
   Die Rate des freigegebenen Glyzerins wird im Verhältnis zur Rate aller Lipide angegeben, die durch Turbidimetrie dosiert wurden.
   Die Prüfmass-Substanzen sind Theophyllin und Isoprenalin.
4) Ergebnisse
   Jeder Extrakt wurde im Vergleich zu Bezugssubstanzen (Theophyllin und Isoprenalin) auf 2 bis 5 verschiedenen Präparationen menschlicher Adipozyten getestet.

Die Ergebnisse werden im Durchschnittswert des Aktivierungs-Prozentsatzes im Verhältnis zu einem Prüfmass-Posten (ohne Cecropia-Extrakt) ausgedrückt.

| Getesteter Extrakt | Konzentration | Anzahl d. Versuche | % Aktivierung d. Lipolyse im Verhältnis zum Prüfmass ohne Extrakt (Durchschnitt) |
|---|---|---|---|
| Cecropia-Extrakt, nach Beispiel 1 präpariert | 0,04 % Gew/V | 5 | + 41 |
| | 0,08 % Gew/V | 5 | + 53 |
| Cecropia-Extrakt, nach Beispiel 3 präpariert | 0,05 % Gew/V | 5 | + 44 |
| | 0,1 % Gew/V | 5 | + 134 |
| Cecropia-Extrakt nach Beispiel 5 präpariert | 0,04 % Gew/V | 5 | + 58 |
| | 0,08 % Gew/V | 2 | + 113 |
| Theophyllin | 1 mM | 5 | + 70 |
| Isoprenalin | 0,1 µM | 5 | + 45 |

Die in dieser Tabelle aufgestellten Ergebnisse zeigen, dass die getesteten Cecropia-Extrakte eine Aktivierung der Lipolyse aufweisen, die nahe der Aktivierung und in gewissen Fällen höher als die beim Vorhandensein der Kontrolmittel (Theophyllin 1 mM oder Isoprenalin 0,1 µM) beobachteten ist.
Aus diesem Grund können die Cecropia-Extrakte in dermo-kosmetischen Schlankheitskur-Präparationen verwendet werden, die ermöglichen, den im Fall von Zellulitis gespeicherten Triglyzeridüberschuss zu reduzieren.

### II. Nachweis einer straffenden Wirkung durch ein mengenbestimmtes Mass in vivo beim Menschen, durch eine horizontale Ausdehnungsmessungs-(Extensiometrie) Technik

### 1) Prinzip des Versuchs

Der Versuch besteht im Messen der Verschiebung der Haut nach einer sinusförmigen, ständigen Kraft, die parallel zu ihrer Oberfläche angewandt wird. Es handelt sich um eine Technik der horizontalen Ausdehnungsmessung (Extensiometrie), die mit einer Vorrichtung des Typs ausgeführt wurde, die in der Anmeldung des französischen Patents N° 98 12125 auf den Namen der Antragstellerin beschrieben wird. Die Bearbeitung der Kraft- und Dehnungssignale durch eine Hysteresisellipse ermöglicht die dynamische Spannung oder DSR ("Dynamic Spring Rate") zu berechnen. Die Anwendung eines straffenden Erzeugnisses auf der Oberfläche der Haut wirkt sich durch eine DSR-Steigerung aus, eine Konsequenz, bei ständiger Kraft, einer Verminderung der Dehnung der Haut während der Beanspruchung. Die Versuche werden in standardisierten Bedingungen hinsichtlich der Temperatur und der relativen Feuchtigkeit vorgenommen.

### 2) Hervorhebung der Aktivität

Ein "Patch" wird auf einen 1 cm² grossen, kutanen Bereich auf dem Handrücken eines Freiwilligens geklebt. Fünf Messungen der horizontalen Ausdehnung werden dann auf der nicht behandelten Haut als Kontrollmittel vorgenommen. Danach wird der Placeboträger per 10 Mikrolitern angewandt. Nach 5-minütigem Trocknen dienen fünf Messungen zur Kontrolle des Trägereffektes. Danach wird die Haut mit dem aktiven Stoff behandelt, dessen straffende Wirkung durch fünf letzte Messungen nach weiterer 5-minütiger Wartezeit gemessen wird. Während jedes Schrittes wird eine Durchschnittsmessung von 5 Messungen vorgenommen. Die endgültigen Ergebnisse werden in prozentualer Variation des DSR zwischen einerseits dem durch Wasser erzeugten Effekt und andererseits der Aktivität des aktiven Wirkstoffes angegeben.

### 3) Ergebnisse

Die Cecropia-Extrakte wurden als wässrige Lösung mit einer 1,5 %igen Konzentration unter diesen Bedingungen mit einer gesunden Volontärin (Testperson) getestet.

Der nach dem Beispiel 4 präparierte Cecropia-Extrakt steigert DSR um 25,4 %.

Der nach dem Beispiel 6 präparierte Cecropia-Extrakt steigert DSR um 52 %.

Die Ergebnisse dieser Tests zeigen deutlich die straffende, kutane Wirkung der Cecropia-Extrakte, die in kosmetischen, straffenden Erzeugnissen (für Gesicht und Büste bestimmt), wiederformgebenden Erzeugnissen, Erzeugnissen für die Augen- und Gesichtskonturen, Anti-Falten-Erzeugnissen und Make-up-Erzeugnissen verwendet werden können.

### III) Nachweis einer weichmachenden Wirkung durch eine mengenmässige (quantitative) in vivo-Messung beim Menschen durch eine reibungsmessende (friktiometrische) Technik

### 1) Prinzip des Versuchs

Der Versuch besteht in der Anwendung einer konstanten Kraft auf der Haut durch einen Gleitschuh, der bei kontrollierter Geschwindigkeit und kontrolliertem Druck in Rotation gebracht wird. Der Reibungsmoment wird gemessen. Er ermöglicht den Reibungskoeffizienten des Gleitschuhs auf der Haut zu berechnen. Der Reibungskoeffizient hängt vom Zustand der Hautoberfläche ab, besonders von ihrem Feuchtigkeitsgehalt und ihrer Weichheit. Je wasserhaltiger (hydrierter) und weicher sich die Haut anfühlt, desto mehr steigert sich der Reibungskoeffizient. Die Versuche wurden unter standardisierten Bedingungen hinsichtlich der Temperatur und der relativen Feuchtigkeit vorgenommen.

### 2) Nachweis der Aktivität

Auf einem 9 cm² grossen kutanen Bereich, der sich auf der Antero-Innenseite des Vorderarms des Volontärs befindet, wird eine Reibungsmeter-Messung auf der Haut ohne Behandlung (T0) vorgenommen. Danach wird der Wirkstoff in einer Dosierung von 4 Mikrolitern pro cm² angewandt. Nach 15-minütigem Trocknen dient eine Reibungsmeter-Messung zur Kontrolle der Wirksamkeit des Wirkstoffes (T15). Gleichzeitig wird ein benachbarter, kutaner Bereich als Prüfmass unter den gleichen Bedingungen gemessen. Das Ergebnis wird in dem Unterschied der prozentualen Variation zwischen T0 und T15 des behandelten Bereichs im Verhältnis zum Bereich des Prüfmasses angegeben.

### 3) Ergebnisse

Unter diesen Bedingungen steigert der nach dem Beispiel 2 präparierte Cecropia-Extrakt, der als wässrige Lösung mit einer 1,5 %igen Konzentration auf einer gesunden Volontärin getestet wurde, den Reibungskoeffizienten um 31,5 %.

Das Ergebnis dieses Tests zeigt den Verbesserungseffekt der kutanen Weichheit und der durch die Cecropia-Extrakte gelieferten Feuchtigkeit.

### IV) Nachweis einer Aktivität der Verbesserung des strahlenden Glanzes des Teints durch eine mengenmässige in vivo-Messung beim Menschen durch eine Brillantometrie-Technik

### 1) Prinzip des Versuchs

Der Versuch besteht im Beleuchten der Haut in polarisiertem Licht, danach im Messen des reflektierten Lichts unter verschiedenen Winkeln, die gleichwertig mit denen des einfallenden Lichts sind, das heisst, der vollkommenen Verteilung des reflektierten Lichts je nach dem Winkel. Es handelt sich um eine Goniophotometrie-Technik. Das polarisierte Licht ermöglicht die Spiegelreflexion (oder die glänzende Reflexion) von der ausgestrahlten Reflexion besser zu unterscheiden.

Der Versuch wurde mit dem Verfahren und der Vorrichtung vorgenommen, die in der französischen Patentanmeldung N° 98 12589 auf den Namen der Antragstellerin beschrieben werden.

Das Ergebnis einer Messung auf der Haut wirkt sich durch eine Kurve der Spiegelreflexion nach dem Messwinkel aus. Die Kurve weist einen mehr oder weniger hohen Höchstwert bei 0° auf. Der durchschnittliche Wert des Spiegel-Reflexionsvermögens wird zwischen - 60° und + 60° berechnet. Dieser durchschnittliche Wert ist repräsentativ für den strahlenden Glanz des Teints. Wenn sich sein Wert steigert, verbessert sich der strahlende Glanz des Teints.

Die Versuche wurden unter standardisierten Bedingungen hinsichtlich der Temperatur und der relativen Feuchtigkeit vorgenommen.

### 2) Nachweis der Aktivität

Auf einem 9 cm² grossen, kutanen Bereich, der sich auf der Antero-Innenseite des Vorderarms des Volontärs befindet, wird eine Brillantometrie-Messung auf der Haut ohne Behandlung (T0) vorgenommen. Dann wird der Wirkstoff in der Dosierung von 4 Mikrolitern pro cm² angewandt. Nach 5-minütigem Trocknen dient eine Brillantometrie-Messung zur Kontrolle der Wirksamkeit des Wirkstoffs (T5). Das Ergebnis wird als Unterschied der prozentualen Variation zwischen T0 und T5 auf dem behandelten kutanen Bereich ausgedrückt.

### 3) Ergebnisse

Die Cecropia-Extrakte wurden unter diesen Bedingungen als wässrige Lösung mit einer 1,5 %igen Konzentration auf einer gesunden Volontärin getestet.

Der nach dem Beispiel 3 präparierte Cecropia-Extrakt steigert den durchschnittlichen Wert des Reflexionsvermögens um 11 %.

Der nach dem Beispiel 6 präparierte Cecropia-Extrakt steigert den durchschnittlichen Wert des Reflexionsvermögens um 14 %.

Die Ergebnisse dieser Tests beweisen also den verbessernden Effekt des strahlenden Glanzes des Teints mittels Cecropia-Extrakten, die in kosmetischen, für diesen Gebrauch bestimmten Erzeugnissen Anwendung finden können.

Nach einem Merkmal der Erfindung enthalten die verschiedenen vorab erwähnten kosmetischen Zusammensetzungen auf vorteilhafte Weise als Wirkungsmittel, alleine oder in Verbindung mit anderen Wirkungsmitteln zwischen, 0,001 % und 20 % im Gewicht, vorzugsweise zwischen 0,1 % und 3 % im Gewicht einen Extrakt oder ein Gemisch aus Extrakten der Pflanzen der Cecropia-Gattung, besonders der Cecropia obtusa und/oder der Cecropia peltata.

Der das Wirkungsmittel bildende Extrakt kann in jeglicher galenischer Form angewendet werden, die auf dem Gebiet der Kosmetik verwendbar ist, wie zum Beispiel Emulsionen (Ö/W und W/Ö), Gesichtswasser, Milch, Gele, Hydrogele, Cremes, Pommaden, Seifen, Stäbchen, Sprühmittel, Haarwasser und Shampoos.

Ausserdem kann der das Wirkungsmittel bildende Extrakt in einen oder mehrere kosmetische Vektor/e, besonders einen oder meherere Vektor/e hinzugefügt werden, der/die aus der Gruppe gewählt wird/werden, die durch die Liposome, Makro-Mikro oder Nanokapseln, die Makro-Mikro oder Nanopartikel und andere ähnliche und bekannte Formen gebildet wird.

Zur nicht einschränkenden Veranschaulichung möglicher Ausführungsarten für kosmetische Erzeugnisse oder Zusammensetzungen nach der Erfindung werden nachstehend verschiedene Formulierungen von kosmetischen Zusammensetzungen angegeben, die einen oder mehrere Extrakt/e von Pflanzen der Cecropia-Gattung enthalten.

### Beispiel 1

Ein kosmetisches Erzeugnis in der Form einer Schlankheitscreme für den Körper nach der Erfindung kann zum Beispiel eine Gewichtszusammensetzung aufweisen, die von den folgenden Fraktionen A, B und C ausgehend gebildet wird:
Fraktion A
   - Glyzerinstearat und Ceteareth 20 und Ceteareth 10 und ketylisches Palmitat 5,0 %
   - ketostearilischer Alkohol 2,0 %
   - Decyloleat 3,0 %
   - Paraffinöl 3,0 %
   - Karitebutter 3,0 %
Fraktion B
   - Glyzerin 3,0 %
   - hydrolisierte Weizenproteine 0,5 %
   - Cecropia-Ethanolische Extrakte nach dem Beispiel 5 des Verfahrens 2,0 %
   - Wasser 78,3 %
Fraktion C
   - Parfum 0,2 %

Der Herstellungsvorgang besteht in der getrennten Vorbereitung der Fraktionen A und B unter Rühren bei 80° C, im Hinzufügen der Fraktion A in die Fraktion B unter Turbinenrühren, danach in der Abkühlung des Gemischs auf Raumtemperatur und schliesslich dem Hinzufügen der Fraktion C.

### Beispiel 2

Ein kosmetisches Erzeugnis in der Form eines Schlankheitsgels für den Körper nach der Erfindung kann zum Beispiel eine Gewichtszusammensetzung aufweisen, die von den folgenden Fraktionen A, B, C, D, E und F ausgehend gebildet wird:
Fraktion A
   - destilliertes Wasser 49,95 %
   - Elastab 50J (Laboratoires Sérobiologiques) 0,5 %
   - Carrageenan 0,1 %
Fraktion B
   - Karbomer 0,35 %
   - destilliertes Wasser 31,95 %
Fraktion C
   - Propylen-Glykol 2,00 %
   - Dimethikon-Copolyol 3,00 %
Fraktion D
   - 20 %iges Triethanolamin in wässriger Lösung 2,1 %
Fraktion E
   - Kathon CG (der Firma Rohm u. Haas) 0,05 %
Fraktion F
   - 50 %iger Cecropia-Ethanolextrakt nach dem Beispiel 3 des Verfahrens 1,0 %
   - destilliertes Wasser 9,0 %

Der Herstellungsvorgang des vorab erwähnten Gels besteht hauptsächlich in der getrennten Vorbereitung der Fraktionen A und B bei 75 ° C unter Turbinenrühren, danach in ihrer Abkühlung auf Raumtemperatur und danach in der Vorbereitung der Fraktion F durch Dispersion des Extraktes in seinem zehnfachen Gewicht in Wasser und im allmählichen Hinzufügen der Fraktionen B, C, D, E und F bei Raumtemperatur und unter Turbinenrühren und schliesslich im Fortfahren des Planetenrührens bis zur Homogenisierung.

## Patentansprüche

1. Nichttherapeutische Verwendung mindestens eines Extraktes einer Pflanze, die zur Cecropia-Gattung gehört, als Wirkungsmittel mit einer aktivierenden Wirkung auf die Lipolyse in einer kosmetischen Zubereitung für eine äussere lokale Anwendung für die Haut, die Schleimhäute und/oder die Epithel- oder Körperanhanggebilde.

2. Verwendung mindestens eines Extraktes einer Pflanze, die zur Cecropia-Gattung gehört, nach Anspruch 1 als Wirkungsmittel mit schlankheitsfördernden Eigenschaften in einer kosmetischen Zubereitung.

3. Verwendung mindestens eines Extraktes einer Pflanze, die zur Cecropia-Gattung gehört, nach Anspruch 1 und/oder 2 als straffendes Wirkungsmittel in einer kosmetischen Zubereitung.

4. Verwendung mindestens eines Extraktes einer Pflanze, die zur Cecropia-Gattung gehört, nach mindestens einem der Ansprüche 1 bis 3, als Wirkungsmittel zur Verbesserung des strahlenden Glanzes des Teints in einer kosmetischen Zubereitung.

5. Verwendung mindestens eines Extraktes einer Pflanze, die zur Cecropia-Gattung gehört, nach mindestens einem der Ansprüche 1 bis 4, als weichmachendes Wirkungsmittel in einer kosmetischen Zubereitung.

6. Verwendung mindestens eines Extraktes einer Pflanze, die zur Cecropia-Gattung gehört, zur Herstellung einer dermopharmazeutischen Zubereitung für die Aktivierung der Lipolyse, wobei diese Zubereitung für eine äussere lokale Anwendung für die Haut, die Schleimhäute und/oder die Epithel- oder Körperanhanggebilde bestimmt ist.

7. Verwendung nach mindestens einem der Ansprüche 1 bis 6, **dadurch gekennzeichnet, dass** das Wirkungsmittel gebildet wird aus Extrakten der Pflanze Cecropia obtusa und/oder Cecropia peltata.

8. Verwendung nach mindestens einem der Ansprüche 1 bis 7, **dadurch gekennzeichnet, dass** das Wirkungsmittel gebildet wird aus Extrakten der Pflanzenteile ausgewählt aus der Gruppe, die gebildet wird aus Wurzeln, Rinden, Blättern, beblätterten Stengeln, Blüten, Früchte und/oder Körner.

9. Verwendung nach mindestens einem der Ansprüche 1 bis 8 **dadurch gekennzeichnet, dass** der das Wirkungsmittel bildende Extrakt aus einer Fraktion gebildet wird, die rohe, halb gereinigte oder gereinigte Polysaccharide enthält.

10. Verwendung nach mindestens einem der Ansprüche 1 bis 9, **dadurch gekennzeichnet, dass** der das Wirkungsmittel bildende Extrakt in Mengen zwischen 0,001 % und 20 % Gewicht bezogen auf die Zubereitung eingezetzt wird.

11. Verwendung nach mindestens einem der Ansprüche 1 bis 9, **dadurch gekennzeichnet, dass** der das Wirkungsmittel bildende Extrakt in Mengen zwischen 0,1 % und 3 % Gewicht bezogen auf die Zubereitung eingesetzt wird.

12. Verwendung nach mindestens einem der Ansprüche 1 bis 11, **dadurch gekennzeichnet, dass** der das Wirkungsmittel bildende Extrakt in einen oder mehrere Vektor/e der/die aus der von Liposomen, Makro-Mikro- oder Nanokapseln, Makro- Mikro-, oder Nanopartikeln und anderen ähnlichen Formen gebildeten Gruppe ausgewählt ist/sind, inkorporiert ist.

## Claims

1. Non-therapeutic use of at least one extract of a plant of the genus Cecropia as an active principle with an activating effect on lipolysis in a cosmetic preparation for external local application to the skin, the mucosa and/or the epithelial or body appendages.

2. Use of at least one extract of a plant of the genus Cecropia as claimed in claim 1 as an active principle with slimming properties in a cosmetic preparation.

3. Use of at least one extract of a plant of the genus Cecropia as claimed in claim 1 and/or 2 as a tightening active principle in a cosmetic preparation.

4. Use of at least one extract of a plant of the genus Cecropia as claimed in at least one of claims 1 to 3 as an active principle for improving the radiance of the complexion in a cosmetic preparation.

5. Use of at least one extract of a plant of the genus Cecropia as claimed in at least one of claims 1 to 4 as a softening active principle in a cosmetic preparation.

6. Use of at least one extract of a plant of the genus Cecropia for the production of a dermopharmaceutical preparation for activating lipolysis, this preparation being intended for external local application to the skin, the mucosa and/or the epithelial or body appendages.

7. Use claimed in at least one of claims 1 to 6, **characterized in that** the active principle is formed from extracts of the plant Cecropia obtusa and/or Cecropia peltata.

8. Use claimed in at least one of claims 1 to 7, **characterized in that** the active principle is formed from extracts of plant parts selected from the group consisting of roots, bark, leaves, leaf-covered stems, flowers, fruits and/or seeds.

9. The use claimed in at least one of claims 1 to 8, **characterized in that** the extract forming the active principle is formed from a fraction containing crude, semi-purified or purified polysaccharides.

10. Use claimed in at least one of claims 1 to 9, **characterized in that** the extract forming the active principle is used in quantities of 0.001% to 20% by weight, based on the preparation.

11. Use claimed in at least one of claims 1 to 9, **characterized in that** the extract forming the active principle is used in quantities of 0.1% to 3% by weight, based on the preparation.

12. Use claimed in at least one of claims 1 to 11, **characterized in that** the extract forming the active principle is incorporated in one or more vectors(s) selected from the group consisting of liposomes, macro-, micro- or nanocapsules, macro-, micro- or nanoparticles and other similar forms.

## Revendications

1. Utilisation non thérapeutique d'au moins un extrait d'une plante appartenant au genre Cecropia, comme agent actif ayant une action d'activation sur la lipolyse dans une préparation cosmétique, en vue d'une application locale externe pour la peau, les muqueuses et/ou l'épithélium ou les phanères.

2. Utilisation d'au moins un extrait d'une plante appartenant au genre Cecropia selon la revendication 1 comme agent actif ayant des propriétés amincissantes dans une préparation cosmétique.

3. Utilisation d'au moins un extrait d'une plante appartenant au genre Cecropia, selon la revendication 1 et/ou 2, comme agent actif tenseur dans une préparation cosmétique.

4. Utilisation d'au moins un extrait d'une plante appartenant au genre Cecropia, selon au moins une des revendications 1 à 3,
comme agent actif pour améliorer l'éclat rayonnant du teint dans une préparation cosmétique.

5. Utilisation d'au moins un extrait d'une plante appartenant au genre Cecropia, selon au moins une des revendications 1 à 4,
comme agent actif assouplissant dans une préparation cosmétique.

6. Utilisation d'au moins un extrait d'une plante appartenant au genre Cecropia pour la production d'une préparation dermo-pharmaceutique visant à l'activation de la lipolyse, cette préparation étant destinée à une application locale externe pour la peau, les muqueuses et/ou l'épithélium ou les phanères.

7. Utilisation selon au moins une des revendications 1 à 6,
**caractérisée en ce que**
l'agent actif est formé à partir d'extraits de la plante Cecropia obtusa et/ou Cecropia peltata.

8. Utilisation selon au moins une des revendications 1 à 7,
**caractérisée en ce que**
l'agent actif est formé à partir d'extraits des parties de plantes choisies dans le groupe formé par les racines, les écorces, les feuilles, les tiges revêtues de feuilles, les fleurs, les fruits et/ou les graines.

9. Utilisation selon au moins une des revendication 1 à 8,
**caractérisée en ce que**
l'extrait formant l'agent actif est formé à partir d'une fraction qui contient des polysaccharides bruts, semi-purifiés ou purifiés.

10. Utilisation selon au moins une des revendications 1 à 9,
**caractérisée en ce que**
l'extrait formant l'agent actif est utilisé en des quantités comprises entre 0,001 % et 20 % en poids par rapport à la préparation.

11. Utilisation selon au moins une des revendications 1 à 9,
**caractérisée en ce que**
l'extrait formant l'agent actif est utilisé en des quantités comprises entre 0,1 % et 3 % en poids par rapport à la préparation.

12. Utilisation selon au moins une des revendications 1 à 11,
**caractérisée en ce que**
l'extrait formant l'agent actif est incorporé dans un ou plusieurs vecteur(s) choisi(s) dans le groupe formé par les liposomes, les macro-, micro- ou nanocapsules, les macro-, micro- ou nanoparticules et d'autres formes analogues.
